# EUROPEAN PATENT APPLICATION

(11) **EP 4 512 310 A1**
(43) Date of publication of application: **26.02.2025**
(21) Application number: 24193300.1
(22) Date of filing: 07.08.2024
(51) Int. Cl.: A61B 5/00, A61B 5/145

(54) **MICRONEEDLE-INTEGRATED INTERSTITIAL FLUID BIOMARKER SENSOR SYSTEMS AND METHODS THEREOF**

(30) Priority: 25.08.2023 US 202318456193
(71) Applicant: General Electric Company, Schenectady, NY 12345 (US)
(72) Inventor: BURNS, Andrew Arthur Paul, Niskayuna, 12309 (US); ALIZADEH, Azar, Niskayuna, 12309 (US); LENIGK, Ralf, Niskayuna, 12309 (US)
(74) Representative: Kilburn & Strode LLP

(57) **Abstract**

A wearable interstitial fluid (ISF) sensing includes a first patch layer (102) and an electronics module component layer (106). The electronics module component layer (106) is laminated to the first patch layer (102) to form a patch component (112) and a plurality of microneedles (114) extending from the patch component (112). The electronics module component layer (106) includes a substrate (168) and a sensor positioned on the substrate (168). Each microneedle of the plurality of microneedles (114) includes a microneedle base (146) extending from the patch component (112), a body (148) extending from the microneedle base (146), and a tip (150) extending from the body (148). The body (148) defines a slot (160) extending through the first patch layer (102) of the body (148). The slot (160) defines a first opening (212) and a second opening (214). The at least one sensor (144) is positioned over the second opening (214). The first opening (212), the slot (160), and the sensor define a channel (210) exposed to the environment. The channel (210) is configured to channel (210) ISF to the sensor (144).

## Description

### STATEMENT REGARDING FEDERALLY SPONSORED RESEARCH AND DEVELOPMENT

This invention was made with Government support under a Development Agreement supported by an award by the Air Force Research Laboratory. The Government has certain rights in this invention.

### BACKGROUND

The subject matter described herein relates generally to wearable sensing systems and, more particularly, to wearable systems for sensing biomolecules in the interstitial fluid of a user.

Continuous or on demand monitoring of biochemical data, or biomarkers, may be important for certain medical conditions. For example, monitoring of biomarkers or drugs administered to the user may enable a user being monitored or a medical professional to medically intervene when the biomarkers indicate that medical intervention could be advantageous and/or necessary to properly manage a medical condition. Poor management of these medical conditions may result in short-term or long-term injury to the user. Similarly, biomarkers may be monitored to evaluate the performance of a user during athletic, military, or cognitive activities. The ability to detect and quantify biomarkers in situ and in real-time enables the user or observers to implement changes in the short-term or long-term to augment performance or prevent degradation of performance (e.g., maintaining hydration, ensuring proper rest, increasing or decreasing training/task load, administering of pharmaceuticals or supplements to enhance performance, etc.).

At least some known biochemical monitoring devices monitor the blood of the user. These biochemical monitoring devices typically include at least one needle or microneedle that penetrates the skin of the user and channel the blood to sensing devices that are located on the surface of the skin. The sensing devices detect and report the presence and/or concentration of biomarkers in the blood to the user or the medical professional. However, because the sensing devices are located on the surface of the skin, the blood is typically continually replenished and must be disposed of after the sensing devices have analyzed the blood. Typically, a wick absorbs the blood, and the wick is discarded after the wick cannot absorb any more blood. As such, the wick is typically frequently replaced in order to continually monitor and analyze the blood. Additionally, these biochemical monitoring devices may be difficult for a user to use due to blood clots (e.g., the extracted blood may clot within the biochemical monitoring device). Moreover, these biochemical monitoring devices may be painful for the user to use because the needle extends through innervated layers within the skin.

At least some known biochemical monitoring devices detect and analyze the sweat of the user. These biochemical monitoring devices are completely non-invasive (i.e., the biochemical monitoring devices do not penetrate the skin of the user), are typically applied to the skin of the user, and typically include a patch secured to the skin with an adhesive. Accordingly, these biochemical monitoring devices generally are not painful for the user to wear. A sensing device is attached to the patch that detects biomarkers in the sweat of the user. However, for certain biomarkers, concentrations in sweat are poorly correlated to their concentrations in the blood of the user. As such, sweat detecting biochemical monitoring devices may not report accurate results for certain analytes/medical conditions/performance scenarios. Additionally, because the biochemical monitoring devices detect bodily fluids that are external to the body, the sweat may become contaminated during contact with the skin and, as such, the quality and quantity of the sample may depend on the environment. Finally, these devices rely upon the presence of sweat in sufficient volumes to fill the sensor, and are thus both limited to situations where sufficient sweat is produced, and may be variable in temporal resolution as a function of sweat rate/environment.

In contrast, for many medical conditions and performance metrics, the concentration of the biomarkers in interstitial fluid (ISF) is correlated to the concentration of the biomarkers in the blood of the user because ISF is in continuous equilibrium with blood via the capillary vasculature. Additionally, because ISF is not excreted, ISF is unlikely to be contaminated by the environment. Moreover, ISF, which makes up about 20% of bodily fluid, does not clot, making it an excellent source for continuous and on-demand biomarker monitoring. Furthermore, in-situ monitoring of ISF (monitoring ISF inside the dermis of the user) is inherently a more temperature controlled monitoring technique than other ISF monitoring techniques because temperature fluctuations with the user's body are minimal.

At least some known biochemical monitoring devices monitor the ISF of the user. These biochemical monitoring devices typically include at least one needle or microneedle that penetrates the skin of the user and channels the ISF to sensing devices that are located on the surface of the skin. These sensing devices detect biomarkers in the ISF of the user. However, because the sensing devices are located on the surface of the skin, the ISF is continually replenished and disposed of after the sensing devices have analyzed the ISF. Typically, a wick absorbs the ISF, and the wick is discarded after the wick cannot absorb any more ISF. As such, the wick is typically frequently replaced in order to continually monitor and analyze the ISF. Because of the low hydrostatic pressure of interstitial fluid in tissue, significant force is applied to extract interstitial fluid out of the tissue either as a negative ambient pressure at the skin surface (vacuum) or positive mechanical pressure on the skin to force the ISF out. Both of these scenarios increase the complexity of ISF collection devices, often require direct user intervention, and add to the perceived discomfort of the user.

Furthermore, at least some known biochemical monitoring devices monitor the ISF of the user subcutaneously. These biochemical monitoring devices typically include an applicator with at least one needle that penetrates the skin of the user and deposits at least one sensing device deep within the tissue (e.g., in subcutaneous fat for continuous glucose monitoring). The sensing device is communicatively coupled to electronics positioned supracutaneously that report the detected biomarkers to the user or the medical professional. However, because the sensing device penetrates the skin, the cells adjacent the sensing device may be irritated by the sensing device, and, as such, may produce ISF proximate the sensing device that could distort the concentrations of certain inflammation-related biomarkers detected by the sensing device.

### BRIEF DESCRIPTION

In one aspect, a wearable interstitial fluid (ISF) sensing device is provided. The wearable ISF sensing device includes a first patch layer and an electronics module component layer. The electronics module component layer is laminated to the first patch layer to form a patch component and a plurality of microneedles extending from the patch component. The electronics module component layer includes a substrate and at least one sensor positioned on the substrate. Each microneedle of the plurality of microneedles includes a microneedle base extending from the patch component, a body extending from the microneedle base, and a tip extending from the body. The body defines a slot extending through the first patch layer of the body. The slot defines a first opening and a second opening. The at least one sensor is positioned over the second opening. The first opening, the slot, and the at least one sensor define a channel exposed to the environment. The channel is configured to channel ISF to the at least one sensor.

In another aspect, a method of detecting a biomarker within the interstitial fluid (ISF) of a user is provided. The method includes adhering a wearable ISF sensing device to the user's skin. The wearable ISF sensing device includes at least one microneedle and at least one sensor positioned within the microneedle. The at least one microneedle includes a microneedle base, a body extending from the microneedle base, and a tip extending from the body. The body defines a slot extending through the body. The slot defines a first opening and a second opening. The at least one sensor is positioned over the second opening. The first opening, the slot, and the at least one sensor define a channel. The method also includes channeling the ISF into the channel to the at least one sensor. The method further includes detecting at least one biomarker with the at least one sensor.

### DRAWINGS

These and other features, aspects, and advantages of the present disclosure will become better understood when the following detailed description is read with reference to the accompanying drawings in which like characters represent like parts throughout the drawings, wherein:
FIG. 1 is a plan view of an exemplary wearable ISF sensing module.
FIG. 2 is an exploded perspective view of the wearable ISF sensing module shown in FIG. 1.
FIG. 3 is a plan view of a top patch layer of the wearable ISF sensing module shown in FIG. 1.
FIG. 4 is a plan view of a bottom patch layer of the wearable ISF sensing module shown in FIG. 1.

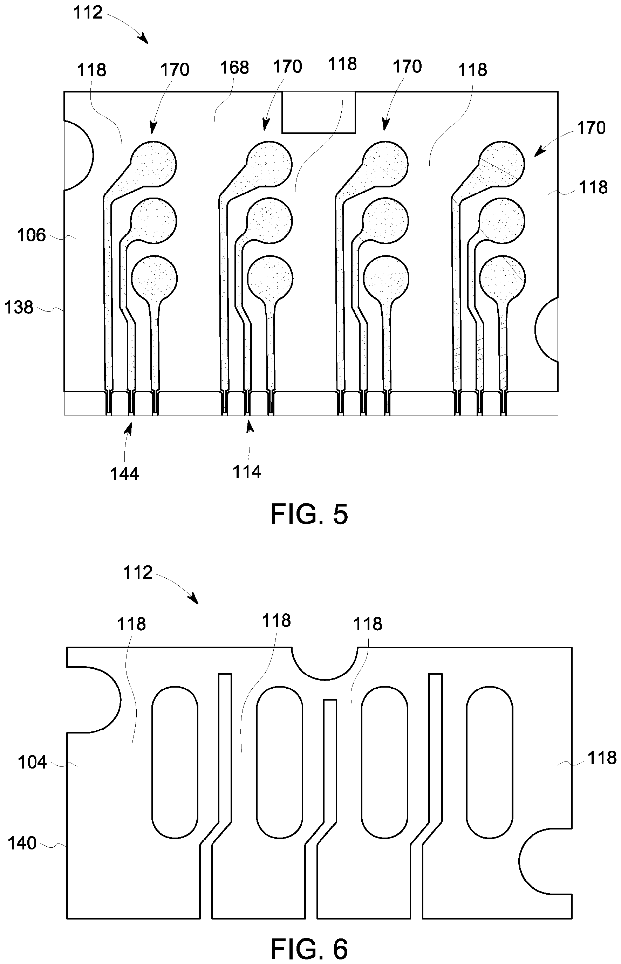

FIG. 5 is a plan view of an electronics module component layer of the wearable ISF sensing module shown in FIG. 1.
FIG. 6 is a plan view a first adhesive layer of the wearable ISF sensing module shown in FIG. 1.
FIG. 7 is a plan view of a second adhesive layer of the wearable ISF sensing module shown in FIG. 1.
FIG. 8 is an exploded perspective view of a microneedle of the wearable ISF sensing module shown in FIG. 1.
FIG. 9 is a perspective view of the top patch layer of the microneedle module in FIG. 8.
FIG. 10 is a plan view of the top patch layer of the microneedle shown in FIG. 8.

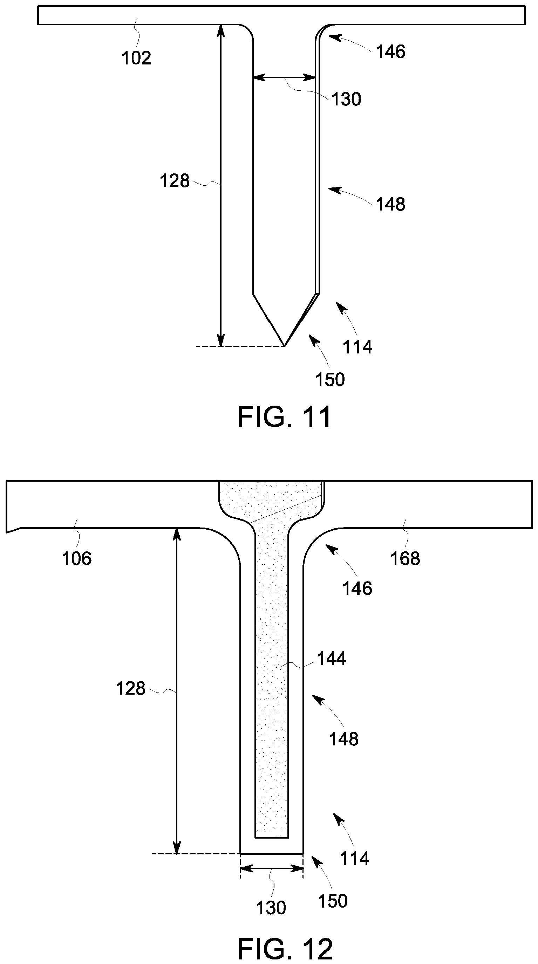

FIG. 11 is a plan view of the bottom patch layer of the microneedle shown in FIG. 8.
FIG. 12 is a plan view of the electronics module component layer of the microneedle shown in FIG. 8.
FIG. 13 is a block diagram of an exemplary electronics architecture.
FIG. 14 is an electrical diagram of a plurality of sensors printed on a substrate of the electronics module component layer shown in FIG. 5.
FIG. 15 is a diagram of the wearable ISF sensing device shown in FIG. 1 positioned on a user's skin with the microneedles shown in FIG. 8 inserted into the dermis of the user's skin.

Unless otherwise indicated, the drawings provided herein are meant to illustrate features of embodiments of the disclosure. These features are believed to be applicable in a wide variety of systems comprising one or more embodiments of the disclosure. As such, the drawings are not meant to include all conventional features known by those of ordinary skill in the art to be required for the practice of the embodiments disclosed herein.

### DETAILED DESCRIPTION

In the following specification and the claims, reference will be made to a number of terms, which shall be defined to have the following meanings.

The singular forms "a", "an", and "the" include plural references unless the context clearly dictates otherwise.

Approximating language, as used herein throughout the specification and claims, may be applied to modify any quantitative representation that could permissibly vary without resulting in a change in the basic function to which it is related. Accordingly, a value modified by a term or terms, such as "about," "substantially," and "approximately," are not to be limited to the precise value specified. In at least some instances, the approximating language may correspond to the precision of an instrument for measuring the value. Here and throughout the specification and claims, range limitations may be combined and/or interchanged, such ranges are identified and include all the sub-ranges contained therein unless context or language indicates otherwise.

The systems and methods described herein provide a wearable interstitial fluid (ISF) sensing device. The device includes a plurality of layers laminated together to form a patch component and at least one microneedle extending from the patch component. The layers include a first patch layer, a first adhesive layer, an electronics module component layer, a second adhesive layer, and a second patch layer. The first and second patch layers structurally support the electronics module component layer, and the first and second adhesive layers laminate the first and second patch layers to the electronics module component layer. Lamination of the layers ensures that the microneedles remain intact as they are inserted into the skin of the user. As such, the microneedles of the wearable ISF sensing devices described herein are structurally stronger than previous microneedles and remain intact as they monitor biomarkers within the user's ISF.

The microneedle includes a microneedle base, a body, and a tip. The microneedle has a substantially flat or planar profile and a slot is defined within the body of the microneedle. The slot has a first opening and a second opening on opposing faces of the microneedle. The electronics module component layer includes a sensor positioned over the second opening such that the sensor and the slot define a channel that channels ISF to the sensor. The microneedle and the sensor are inserted into the user's skin such that the sensor is positioned within the dermis of the user's skin. The ISF flows into the channel from adjacent tissue, and the sensor detects and measures biomarkers within the ISF. The user, observer, trainer, coach, and/or a medical professional may then monitor a medical condition or a performance/readiness metric based on the data collected by the sensor.

Because the sensors are embedded in the dermis, the ISF that flows to the sensors is continually equilibrated with the surrounding ISF without requiring mechanical mechanisms to replenish the ISF. Additionally, embedding the sensors in the dermis enables the wearable ISF sensing device to detect biomarkers without drawing fluid out of the body of the user, reducing the cleanup and disposal of bodily fluids as the result of biomarker detection and measurement. Moreover, capillary pressure and the compressive force generated during insertion will cause ISF to flow into the channel such that a fluid motive device, such as a vacuum pump or mechanical compression, is not required to replenish the ISF at the sensors. After insertion, passive diffusion from the ISF and equilibration with the ISF inside the channel replenishes the ISF at the sensors. Thus, the wearable ISF sensing device enables detection of biomarkers while reducing cleanup and disposal of bodily fluids and reducing the equipment required to measure biomarkers within the ISF. Additionally, directly measuring ISF in the interstitial space reduces the lag time between changes in biomarker concentration and read-out.

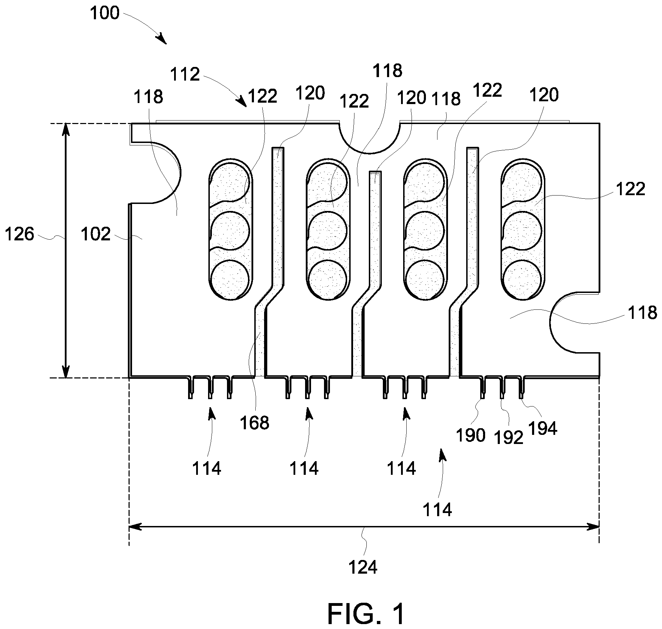

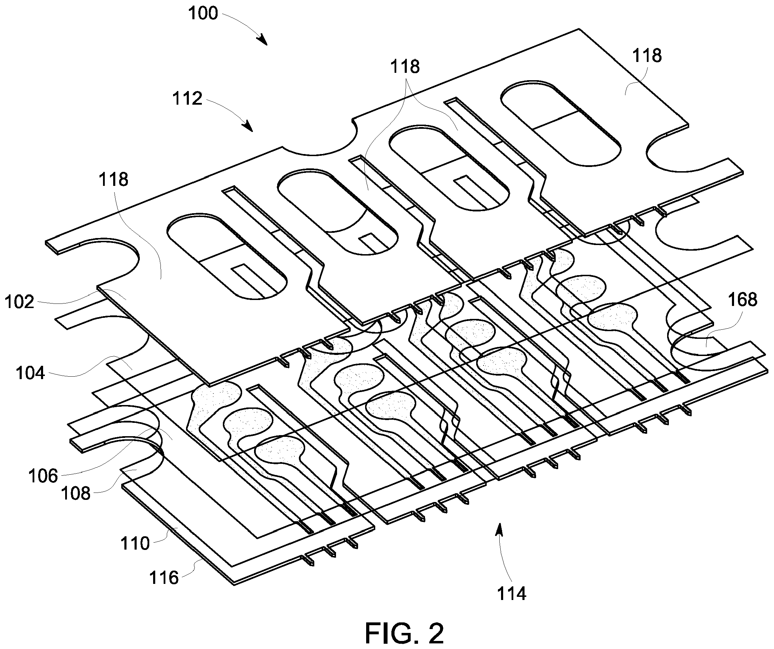

Referring now to the figures, FIG. 1 is a plan view of an exemplary embodiment of a wearable ISF sensing module 100 and FIG. 2 is an exploded perspective view of wearable ISF sensing module 100. As shown in FIGS. 1 and 2, wearable ISF sensing module 100 is formed of a plurality of layers 102-110 laminated together to form a patch component 112 and a plurality of microneedles 114 integrally formed with patch component 112 for insertion into the skin of the user and sensing the ISF of the user, as described in detail below. Patch component 112 supports microneedles 114 during insertion into the skin of the user and adheres wearable ISF sensing module 100 to the skin of the user, as described below. Microneedles 114 detect and measure at least one biomarker within the ISF of the user, enabling wearable ISF sensing module 100 to monitor a medical condition and/or performance metric based on the data collected by microneedles 114. In the exemplary embodiment, wearable ISF sensing module 100 is disposable (i.e., wearable ISF sensing module 100 is generally used once). In an alternative embodiment, at least a portion of wearable ISF sensing module 100 may be reusable (i.e., wearable ISF sensing module 100 may be used for multiple uses).

Wearable ISF sensing module 100 includes a top patch layer or first patch layer 102, a first adhesive layer 104, an electronics module component layer 106, a second adhesive layer 108, and a bottom patch layer or second patch layer 110 laminated together to form wearable ISF sensing module 100. Adhesive layers 104 and 108 attach top patch layer 102, electronics module component layer 106, and bottom patch layer 110. Top patch layer 102 and bottom patch layer 110 structurally support electronics module component layer 106, and electronics module component layer 106 senses the ISF of the user and reports the collected data to the user and/or a medical professional as described herein. Additionally, microneedles 114 are sized and shaped to enable the ISF of the user to flow to electronics module component layer 106 to enable electronics module component layer 106 to detect the ISF.

As shown in FIG. 2, top patch layer 102, first adhesive layer 104, electronics module component layer 106, second adhesive layer 108, and bottom patch layer 110 are arranged in a layered configuration and laminated. Bottom patch layer 110 is the base layer that is attached to the user's skin. Second adhesive layer 108 is applied on top of bottom patch layer 110 and adheres bottom patch layer 110 to electronics module component layer 106 such that second adhesive layer 108 is positioned between bottom patch layer 110 and electronics module component layer 106. First adhesive layer 104 is applied on top of electronics module component layer 106 and adheres electronics module component layer 106 to top patch layer 102 such that first adhesive layer 104 is positioned between top patch layer 102 and electronics module component layer 106. In the exemplary embodiment, first adhesive layer 104 and second adhesive layer 108 are curable adhesives that are positioned between top patch layer 102, electronics module component layer 106, and bottom patch layer 110 as described above. Specifically, in the exemplary embodiment, first adhesive layer 104 and second adhesive layer 108 are sheets of curable adhesives that are positioned between top patch layer 102, electronics module component layer 106, and bottom patch layer 110 as described above. Wearable ISF sensing module 100 is laminated by heating first adhesive layer 104 and second adhesive layer 108 until first adhesive layer 104 and second adhesive layer 108 are thermocured, bonding top patch layer 102, electronics module component layer 106, and bottom patch layer 110.

In an alternative embodiment, first adhesive layer 104 and second adhesive layer 108 are pressure sensitive adhesives that are applied to top patch layer 102 and bottom patch layer 110. Wearable ISF sensing module 100 is laminated by pressing top patch layer 102 and first adhesive layer 104 to electronics module component layer 106 and bottom patch layer 110 and second adhesive layer 108 to electronics module component layer 106 such that the pressure sensitive adhesives bond top patch layer 102, electronics module component layer 106, and bottom patch layer 110 together.

In another alternative embodiment, wearable ISF sensing module 100 does not include first adhesive layer 104 and second adhesive layer 108. Rather, top patch layer 102, electronics module component layer 106, and bottom patch layer 110 are mechanically laminated together. For example, top patch layer 102, electronics module component layer 106, and bottom patch layer 110 are arranged in a layer configuration similar to the layer configuration illustrated in FIG. 2, and mechanical fasteners (not shown) couple top patch layer 102, electronics module component layer 106, and bottom patch layer 110 together.

Patch component 112 of bottom patch layer 110 is attached to the user's skin using an adhesive surface 116 or foam. Alternatively, in some embodiments, patch component 112 may be integrated into clothing and held in place using a suction device or compression clothing. Patch component 112 of bottom patch layer 110 may be affixed to various portions of the user's body, including, but not limited to, the torso, legs, back, neck, arms, etc. For example, patch component 112 of bottom patch layer 110 may be placed on the lower back (e.g., above the waistline, below the rib cage, and adjacent to the spine on either side), on the upper back (e.g., above or on the scapula), on the chest (e.g., below the pectoral muscle or centrally over the sternum), on the lower leg (e.g., over the gastrocnemius or on the lateral face over the tibialis anterior muscle), or on the upper leg (e.g., on the inner thigh).

In the exemplary embodiment, patch components 112 of top patch layer 102 and bottom patch layer 110 are formed of a pliable material, such as, but not limited to, a pliable metal, a pliable plastic, and/or any other material that enables wearable ISF sensing module 100 to operate as described herein. In some embodiments, patch components 112 of top patch layer 102 and bottom patch layer 110 are formed by additively manufacturing, casting, and/or molding a pliable metal or plastic into patch components 112 of top patch layer 102 and bottom patch layer 110. In other embodiments, patch components 112 of top patch layer 102 and bottom patch layer 110 are formed by machining (e.g., laser cutting) pliable metal or plastic into patch components 112 of top patch layer 102 and bottom patch layer 110.

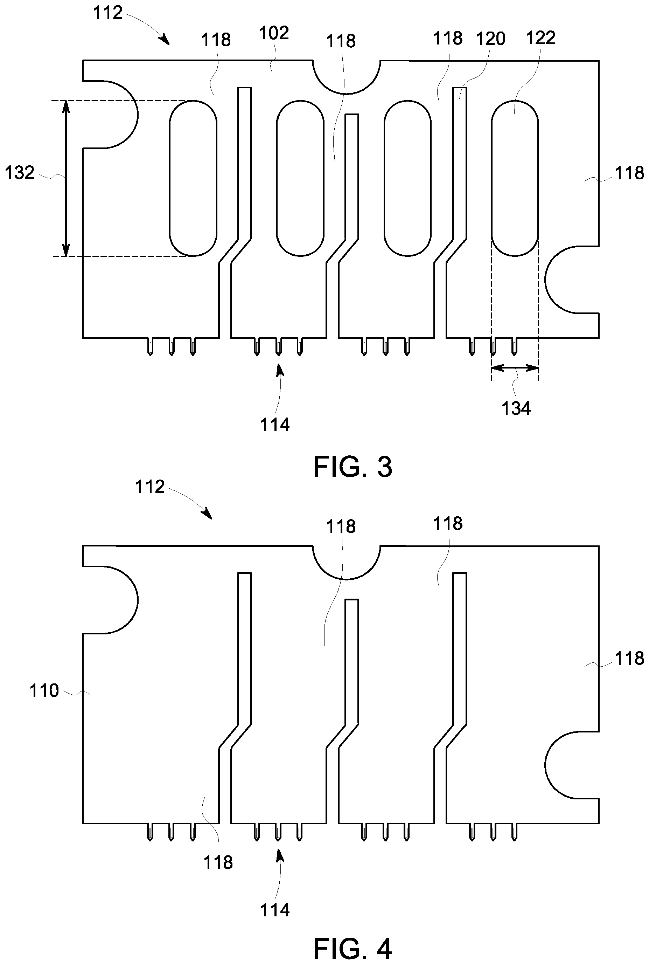

Wearable ISF sensing module 100 includes a plurality of wearable ISF sensing modules 118 that are separable from each other such that each wearable ISF sensing modules 118 operates independently of the other wearable ISF sensing modules 118. In the illustrated embodiment, wearable ISF sensing module 100 includes four wearable ISF sensing modules 118 that are separable from each other. In alternative embodiments, wearable ISF sensing module 100 may include any number of wearable ISF sensing modules 118 that enable wearable ISF sensing module 100 to operate as described herein. Wearable ISF sensing modules 118 are separated from each other after layers 102-110 have been laminated together to form wearable ISF sensing module 100. In some embodiments, wearable ISF sensing modules 118 are separated from each other prior to being shipped to the user. In alternative embodiments, wearable ISF sensing modules 118 are separated from each other by the user and/or medial professional after wearable ISF sensing module 100 is shipped to the user. As shown in FIGS. 1-3 and 7, top patch layer 102 and bottom patch layer 110 define a plurality of separation slots 120 between wearable ISF sensing modules 118 and enable wearable ISF sensing modules 118 to be separated from each other.

Patch component 112 of top patch layer 102 includes a plurality of cut outs 122 that enable the user and/or medical professional to access electronics module component layer 106 positioned between top patch layer 102 and bottom patch layer 110. Specifically, as shown in FIGS. 1-3, cut outs 122 are formed such that portions of electronics module component layer 106 are accessible by the user and/or medical professional. Cut outs 122 are sized and shaped to allow the user, the medical professional, a battery, and/or an electronic device to access electronics module component layer 106. In the exemplary embodiment, cut outs 122 have an oval shape. However, cut outs 122 may have any shape that enables wearable ISF sensing module 100 to operate as described herein.

In the exemplary embodiment, patch component 112 may have a patch component length 124 of approximately 25 millimeters (mm) and a patch component height 126 of approximately 13 mm, microneedles 114 may have a microneedle length 128 of approximately 800 micrometers (µm) and a microneedle width 130 of approximately 100 µm, and cut outs 122 may have a cut out length 132 of approximately 7.5 mm and a cut out width 134 of approximately 2.25 mm. Alternatively, patch component 112, microneedles 114, and/or cut outs 122 may have any dimensions that enable wearable ISF sensing module 100 to function as described herein. Additionally, top patch layer 102 and bottom patch layer 110 each have a patch layer thickness 136 of approximately 75 µm, electronics module component layer 106 has an electronics module component layer thickness 138 of approximately 25 µm, and first and second adhesive layers 104 and 108 each have an adhesive layer thickness 140 of approximately 25 µm such that patch component 112 and microneedles 114 have a thickness 142 of approximately 325 µm.

In the exemplary embodiment, patch component 112 has a rectangular shape. In alternative embodiments, patch component 112 may have any shape that enables wearable ISF sensing module 100 to operate as described herein including, without limitation, an elliptical shape, an elongated shape, a circular shape, and/or any other shape that enables wearable ISF sensing module 100 to operate as described herein. In some instances, the user may want to conceal that they have a certain medical condition and may want to disguise wearable ISF sensing module 100. Accordingly, in some embodiments, wearable ISF sensing module 100 and patch component 112 may be sized and shaped like a common adhesive bandage, or mounted to an insertion device of appropriate size.

In the illustrated embodiment, wearable ISF sensing modules 118 each include three microneedles 114 attached to patch component 112. In alternative embodiment, wearable ISF sensing modules 118 may include any number of microneedles 114 that enable wearable ISF sensing module 100 to operate as described herein. Specifically, as described in detail below, at least one sensor 144 (i.e., an electrode) is attached to each microneedle 114 to detect biomarkers within the ISF. Some medical conditions may require that a plurality of biomarkers are monitored in order to properly monitor and manage the medical condition. Accordingly, each wearable ISF sensing module 118 may include any number of microneedles 114 and sensors 144 that enable wearable ISF sensing module 100 to properly monitor the medical condition.

Microneedles 114 each include a microneedle base 146, a body 148, and a tip 150. Microneedle base 146 is attached to patch component 112, body 148 is attached to microneedle base 146, and tip 150 is attached to body 148. Wearable ISF sensing module 100 is formed such that microneedle base 146 extends from patch component 112 and is substantially coplanar with patch component 112. Additionally, body 148 extends from microneedle base 146 and tip 150 extends from body 148 such that microneedle base 146, body 148, and tip 150 are substantially coplanar with patch component 112. Microneedles 114 may then be bent to an appropriate angle for insertion into the skin of the user. In an exemplary embodiment, microneedles 114 are bent during the manufacturing process prior to sending wearable ISF sensing module 100 to the user. In alternative embodiments, microneedles 114 are coplanar with patch component 112 when they are shipped to the user, and the user and/or medical professional bends the microneedles 114 prior to insertion into the user's skin. In another alternative embodiment, microneedles 114 remain un-bent and are supported by a microneedle insertion device (not shown) during insertion into the user's skin.

In the exemplary embodiment, microneedles 114 each include a first surface 152, a second surface 154 opposite first surface 152, a third surface 156, and a fourth surface 158 opposite third surface 156. First surface 152 and second surface 154 each have a length that is equal to microneedle length 128 and a width that is equal to microneedle width 130. Third surface 156 and fourth surface 158 each have a length that is equal to microneedle length 128 and a width that is equal to thickness 142. In the exemplary embodiment, first surface 152, second surface 154, third surface 156, and fourth surface 158 each have a flat or planar profile. Thus, microneedles 114 each include at least one surface that has a flat or planar profile. In the exemplary embodiment, microneedles 114 each include four surfaces that each have a flat or planar profile.

In the exemplary embodiment, microneedle base 146 is formed of a pliable material to enable microneedle base 146 to be bent. In some embodiments, patch component 112 and microneedles 114 are formed of the same pliable material. In alternative embodiments, patch component 112 is formed of a first material and microneedles 114 are formed of a second material different than the first material. In yet another alternative embodiment, patch component 112 and microneedles 114 may be formed of a plurality of materials. For example, patch component 112 may be formed of the first material, microneedle base 146 may be formed of the second, pliable material, body 148 may be formed of a third material that is different than the first and second materials, and tip 150 may be formed of a fourth material that is different than the first, second, and third materials.

In the exemplary embodiment, the first, second, third, and fourth materials are the same material. Specifically, in the exemplary embodiment, the first, second, third, and fourth materials may be stainless steel or an alloy of stainless steel. In an alternative embodiment, the first, second, third, and fourth materials may be a polymer. In yet another alternative embodiment, each of the first, second, third, and fourth materials may be selected based on the function of the component they form. For example, the third and fourth materials, forming body 148 and tip 150 respectively, may be a material with a hardness (the measure of the resistance to localized plastic deformation induced by either mechanical indentation or abrasion) greater than the hardness of the second material because body 148 and tip 150 puncture the skin of the user. The second material may be a material that has greater pliability than the first, third, and fourth materials because microneedle base 146 bends.

In the exemplary embodiment, each top patch layer 102 of each microneedle 114 defines a slot 160 extending through microneedle base 146 and body 148. In the illustrated embodiment, slot 160 does not extend into tip 150. In an alternative embodiment, slot 160 only extends through a portion of body 148. In another alternative embodiment, slot 160 extends through microneedle base 146, body 148, and tip 150. However, slot 160 may extend through any portion of each top patch layer 102 of each microneedle 114 that enables wearable ISF sensing module 100 to operate as described herein. Slot 160 has a slot length 162 and a slot width 164. In the exemplary embodiment, slot length 162 is substantially greater than slot width 164, and slot 160 forms a channel that channels ISF to sensors 144 for detection of biomarkers, as described herein. Slot 160 extends through top patch layer 102 of microneedle 114 and, as such, slot 160 has a slot depth 166 approximately equal to patch layer thickness 136 of approximately 75 µm. In alternative embodiments, slot 160 may have any size and shape that enables wearable ISF sensing module 100 to operate as described herein.

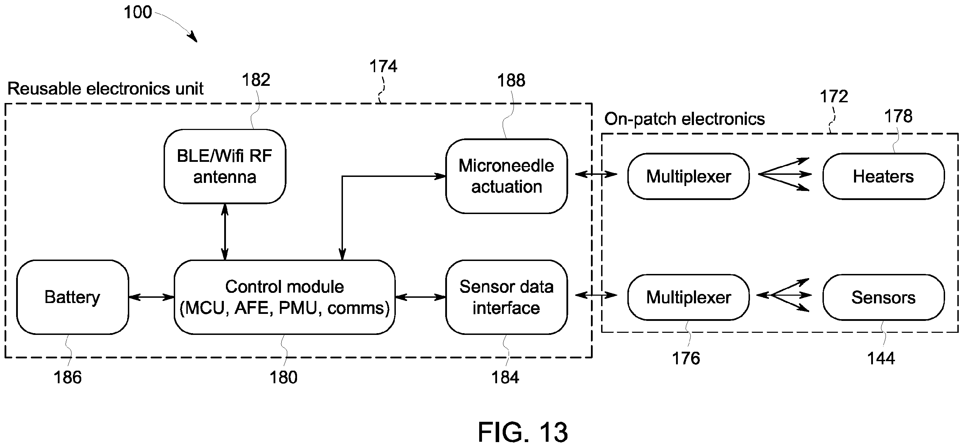

Turning now to electronics module component layer 106 electronics module component layer 106 includes a substrate 168, at least one electrical circuit 170, sensors 144, and communication and energy management components. FIG. 13 is a block diagram of an exemplary electronics architecture of the communication and energy management components. The communication and energy management components described above may include an on-patch control module 172, an off-patch control module 174, and a cable (not shown) communicatively coupling electrical circuit 170, sensors 144, and/or on-patch control module 172 to off-patch control module 174. Electronics module component layer 106 may include any of on-patch control module 172, off-patch control module 174, and the cable. Off-patch control module 174 may include data storage, power management, and communications components, as described herein. On-patch control module 172 may also be referred to herein as an analog-to-digital signal module. Performing the analog to digital conversion close to sensor 144 minimizes noise and spurious signals that may interfere with signals generated by sensors 144, as well as piezoelectric voltages generated in the connection cables. The distributed configuration of electronics module component layer 106 enables reducing the footprint and weight of the portion of electronics module component layer 106 in direct contact with the skin (i.e., on-patch control module 172). Specifically, on-patch control module 172 may be relatively light-weight and have a thin form factors, increasing user comfort and improving robustness of attaching patch component 112 to the user and of wearable ISF sensing module 100 itself. Further, because on-patch control module 172 is separate from off-patch control module 174, off-patch control module 174 may be positioned remotely from patch component 112. Alternatively, on-patch control module 172 and off-patch control module 174 are incorporated in the same component, and are not located remotely from one another. As another alternative, in some embodiments, on-patch control module 172 and off-patch control module 174 are located remotely from one another, but are not communicatively coupled using the cable. Instead, in such embodiments, on-patch control module 172 and off-patch control module 174 communicate with each other wirelessly, using any suitable wireless communications protocol.

Off-patch control module 174 may be positioned remotely from on-patch control module 172 using the cable. For example, off-patch control module 174 may be stored in a pocket of a garment (e.g., pants or shirt), or may be attached, for example, to a belt or a heart-rate monitor strap (e.g., using a clip or hook and loop fasteners). In embodiments where off-patch control module 174 is incorporated into a garment (e.g., an elastic and form-fitting garment), the cable may be a flexible conductor sewn or otherwise incorporated into the garment to allow wearable ISF sensing module 100 to be affixed to a particular location, while off-patch control module 174 is located in an unobtrusive location (e.g., on the back of a collar or on a sleeve of a shirt, over the sternum, on the hem of pants, in a waistband, in a pocket, etc.) or distributed over other portions of the garment.

When wearable ISF sensing module 100 includes both on-patch control module 172 and off-patch control module 174, the cable connects on-patch control module 172 and off-patch control module 174. The cable may be, for example, a headphone audio extension cord or the like. In the exemplary embodiment, on-patch control module 172 includes amplifiers (e.g., for amplifying signals from sensors 144), voltage reference circuits, and an analog-to-digital converter (ADC)/multiplexer (MUX) 176. Additionally, ADC/MUX 176 may activate an insertion mechanism 178 for inserting microneedles 114 into the user's skin. Accordingly, on-patch control module 172 converts measurements from analog to digital signals before transmitting those signals to off-patch control module 174 in the exemplary embodiment. Alternatively, on-patch control module 172 may include any circuitry that enables on-patch control module 172 to function as described herein. On-patch control module 172 has a small footprint, and is low-weight and low-profile, improving device comfort and wearability, and improving mechanical and electrical reliability of the device during natural body movements because internal mechanical stresses are significantly reduced.

Further, in the exemplary embodiment, off-patch control module 174 includes a memory device and a microcontroller (MCU) 180. Off-patch control module 174 may also include a wireless communications unit 182 (e.g., a Bluetooth unit), a sensor data interface unit 184, a battery unit 186, and a microneedle actuation unit 188. Battery unit 186 may be rechargeable, for example, using induction charging. In some embodiments, memory device 180 may be a microSD card or other data storage device that is removably insertable into or permanently installed in off-patch control module 174.

Off-patch control module 174 may be capable of wireless communication with a remote computing device (e.g., a mobile computing device) using Bluetooth communications or alternative approaches such as direct Wi-Fi. Further, off-patch control module 174 may be capable of bi-directional communication, allowing off-patch control module 174 or the remote computing device to store and recall prior data in the event of a communications breakdown.

In some embodiments, off-patch control module 174 communicates with an associated software application installed on the computing device. The software application may include multiple functions that assist a user in using wearable ISF sensing module 100. The software application may upload all data to a cloud storage system, include algorithms for analyzing trends in the data, and enable users to customize the data analysis. The software application may also enable data (in a raw form or subsequent to analysis) to be displayed on the computing device. For example, in some embodiments, the software application (or off-patch control module 174 itself) may analyze the biochemical data collected by wearable ISF sensing module 100. Further, the software application may generate an alarm when the biomarkers deviate from an expected range. Further, data may be stored on off-patch control module 174 as a backup and downloaded to the computing device upon re-establishment of communications.

In the exemplary embodiment, substrate 168 includes a patch attached to patch component 112 of top patch layer 102 and bottom patch layer 110. Specifically, in the exemplary embodiment, substrate 168 is formed of polyethylene terephthalate (PET) and is attached to patch component 112 of top patch layer 102 and bottom patch layer 110 as described above. Electrical circuit 170, sensors 144, and/or on-patch control module 172 may be printed on substrate 168, and substrate 168 may be attached to patch component 112 of top patch layer 102 and bottom patch layer 110. Additionally, after substrate 168 has been attached to patch component 112 of top patch layer 102 and bottom patch layer 110, at least a portion of substrate may be laminated to patch component 112 of top patch layer 102 and bottom patch layer 110 to maintain a position of electrical circuit 170, sensors 144, and/or on-patch control module 172 on substrate 168 and/or patch component 112 of top patch layer 102 and bottom patch layer 110 during use. In an alternative embodiment, wearable ISF sensing module 100 does not include substrate 168. Rather, electrical circuit 170, sensors 144, and/or on-patch control module 172 may be printed directly on patch component 112 of top patch layer 102 and/or bottom patch layer 110.

In another alternative embodiment, electrical circuit 170, sensors 144, and/or on-patch control module 172 are printed on both sides of substrate 168 and bottom patch layer 110 is sized and shaped like top patch layer 102 such that electrical circuit 170, sensors 144, and/or on-patch control module 172 are accessible on both sides of substrate 168. That is, substrate 168 may have electrical circuit 170, sensors 144, and/or on-patch control module 172 on both sides of substrate 168 such that wearable ISF sensing module 100 is double sided, increasing the monitoring capabilities of wearable ISF sensing module 100. Sensors 144 positioned on opposite sides of substrate 168 may be configured to monitor the same biomarker or different biomarkers.

Electrical circuit 170 communicatively couples sensors 144 to on-patch control module 172 and/or off-patch control module 174. Specifically, electrical circuit 170 may be any circuit that transmits data from sensors 144 to on-patch control module 172 and/or off-patch control module 174.

In the illustrated embodiment, each wearable ISF sensing module 100 includes twelve microneedles 114, and each microneedle 114 includes at least one sensor 144 positioned at least partially on body 148 and tip 150. Each sensor 144 is positioned on body 148 and/or tip 150 such that sensors 144 are embedded in the dermis of the user's skin when microneedles 114 are inserted into the user's skin. In the exemplary embodiment, each sensor 144 includes a bio sensor for sensing a biomarker within the ISF. Specifically, sensors 144 detect and measure metabolites, small molecules, proteins, ions, electrolytes, enzymes, hormones, one or more biochemical parameters, and/or any other biomarker that assists with monitoring and control of a medical condition and/or the performance of an athlete.

In the illustrated embodiment, each wearable ISF sensing modules 118 includes three microneedles 114 and each microneedle 114 includes at least one sensor 144 such that electronics module component layer 106 of each wearable ISF sensing modules 118 includes a first sensor 190, a second sensor 192, and a third sensor 194. In some embodiments, first, second, and third sensors 190, 192, and 194 are the same sensors for detecting and measuring the same biomarkers. More specifically, first, second, and third sensors 190, 192, and 194 may be configured to measure the same biochemical species and report the results (either the data from all three sensors 190, 192, and 194 or an average of all three sensors 190, 192, and 194) to the user and/or medical professional. In alternative embodiments, first, second, and third sensors 190, 192, and 194 are different sensors for detecting and measuring the different biomarkers.

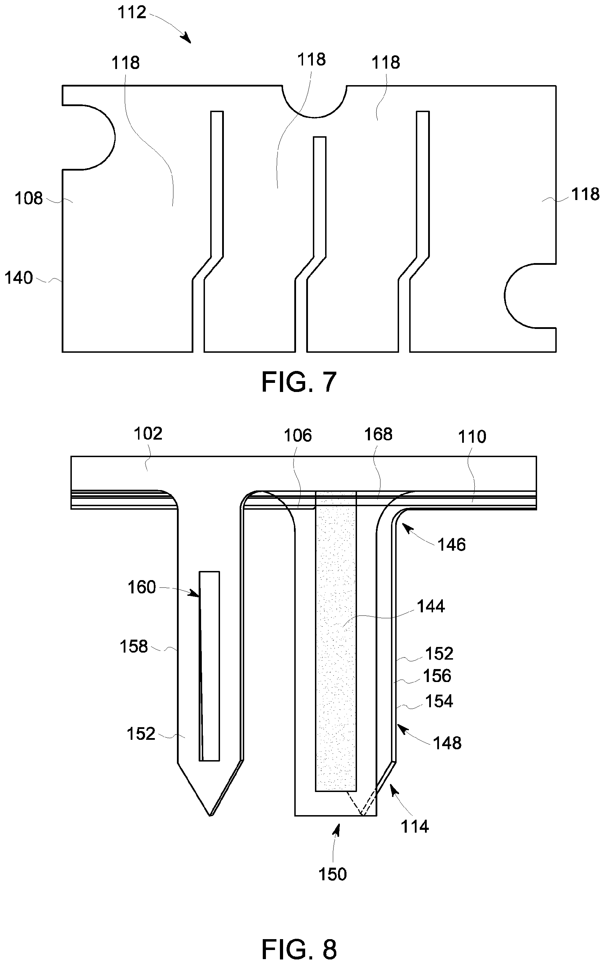

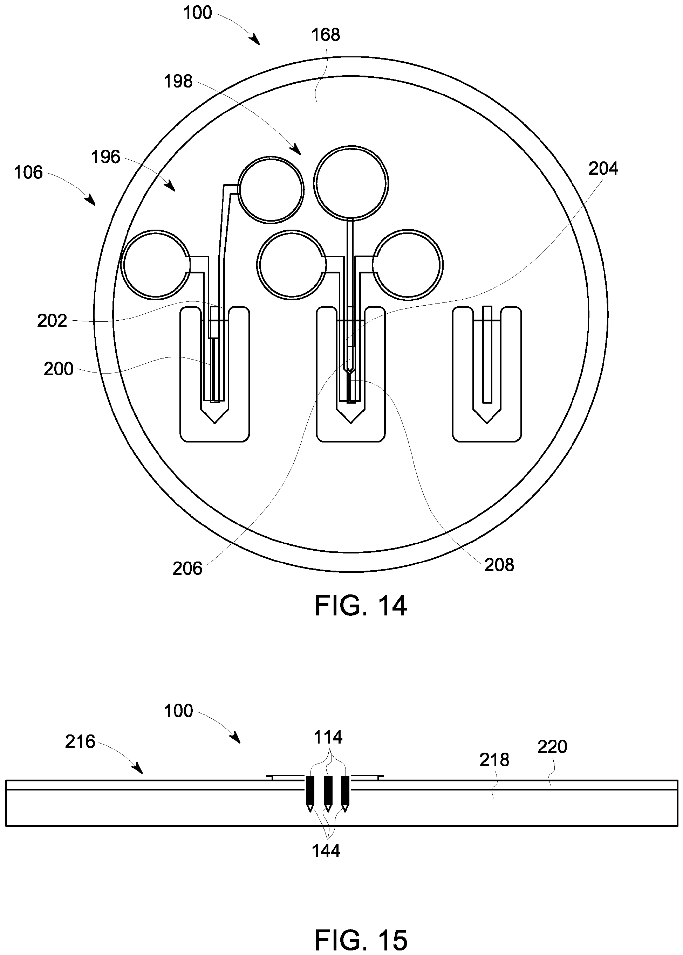

FIG. 14 is an electrical diagram of sensors 144 printed on substrate 168. In the embodiment illustrated in FIG. 7, each sensor 144 includes a 2-electrode and/or a 3-electrode biosensor that detect biomarkers within the ISF. More specifically, electronics module component layer 106 may include a 2-electrode biosensor 196 and/or a 3-electrode biosensor 198. 2-electrode biosensor 196 is a 2-electode system including a working electrode 200 and a counter/reference electrode 202 for detecting a specific biomarker. 3-electrode biosensor 198 is a 3-electode system including a working electrode 204, a reference electrode 206, and a counter electrode 208 for detecting a specific biomarker. 2-electrode biosensor 196 and/or 3-electrode biosensor 198 may detect ISF using a plurality of electroanalytical detection methods including, but not limited to, chronoamperometry, cyclic voltammetry, square wave voltammetry, and/or any other electroanalytical detection method that enables sensors 144 to operate as described herein. Additionally, the biomarker that is to be detected at least partially determines the type of sensor 144 embedded on microneedles 114. Additionally, sensors 144 may include other types and/or classes of biosensors. For example, sensors 144 may include field-effect transistor biosensor (not shown) and/or any other type of sensor that enables wearable ISF sensing module 100 to operate as described herein.

For each of the biosensors described herein, working electrodes 200 and 204 include a biorecognition element (BRE) (not shown). For example, when working electrodes 200 and 204 detect metabolites, the BREs include metabolite sensing enzymes immobilized on a surface (not shown) of working electrodes 200 and 204. The choice of BREs, and their kinetics, are key to enabling continuous equilibration with biomarkers within the ISF, where high binding affinities (necessary to sense low concentrations) often correlate with poor reversibility (multiple sensor use). Antibodies, short peptides, aptamer- and non-natural peptide-based BREs may also be used to achieve target on/off equilibration rates compatible with the kinetics of target biomarker concentration fluctuations in vivo. Aptamer and short peptide BREs, in lieu of more commonly used antibodies, may selectively and specifically recognize protein and hormone biomarkers. The use of aptamers and peptides as recognition elements will decrease the cost and increase the simplicity and efficacy of biosensor fabrication. In particular, peptide and aptamer BREs enable target analyte binding in the close vicinity of the sensor surface (compared to antibodies, which are considerably larger), thus providing an effective means to overcome challenges associated with sensing in conductive biological media, such as ISF. Furthermore, aptamer and peptide BREs are considerably more stable than commonly used antibodies, offering clear advantages for in vivo monitoring in biological fluids. Other examples of BREs include non-natural peptides which offer thermal and enzymatic stabilities.

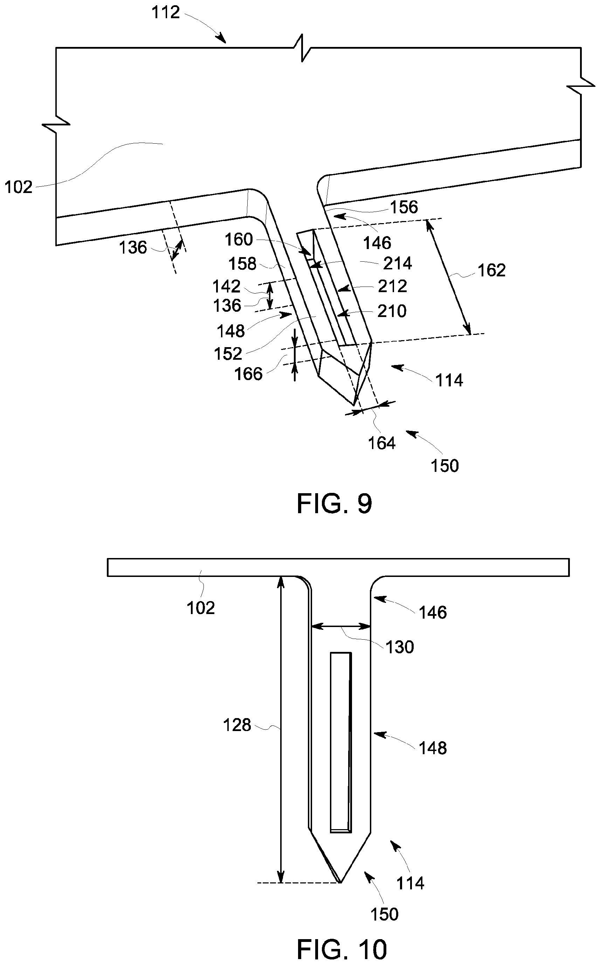

As shown in FIGS. 1, 2, and 8, substrate 168 is positioned on patch component 112 such that electrodes 200, 202, 204, 206, and/or 208 are positioned within slot 160, and substrate 168 and slot 160 define a channel 210 for channeling ISF to electrodes 200, 202, 204, 206, and/or 208. As shown in FIG. 9, slot 160 extends through body 148 of top patch layer 102 forming a first opening 212 and a second opening 214. As shown in FIG. 8, substrate 168 is positioned on patch component 112 such that second opening 214 is covered by substrate 168. First opening 212 remains uncovered and allows ISF to flow into channel 210. More specifically, the pressure of the ISF causes the ISF to flow into channel 210 and to electrodes 200, 202, 204, 206, and/or 208 to be detected and measured. Because microneedles 114 penetrate the skin of the user, the cells adjacent microneedles 114 may be irritated by microneedles 114 and/or sensors 144. The cells proximate sensors 144 may react to the irritation by producing different biomarkers that may alter the ISF proximate microneedles 114. Accordingly, if sensors 144 were located directly adjacent to the irritated cells, the measurements reported by sensors 144 may have an error caused by the reaction of the irritated cells.

The slotted design of microneedles 114 reduces the error caused by the irritated cells. Specifically, electrodes 200, 202, 204, 206, and/or 208 are positioned within slot 160 and channel 210 and are not located directly adjacent to the irritated cells. Additionally, because the ISF flows into channel 210, the ISF detected by electrodes 200, 202, 204, 206, and/or 208 includes ISF surrounding cells that are adjacent to and remote from microneedles 114. Thus, electrodes 200, 202, 204, 206, and/or 208 detect an average biomarker concentration from cells whose position relative to microneedles 114 varies. Moreover, channeling the ISF into channel 210 enables electrodes 200, 202, 204, 206, and/or 208 to detect and measure ISF surrounding different types of cells within the dermis. Thus, the slotted design of microneedles 114 reduces the error caused by the irritated cells by channeling ISF into electrodes 200, 202, 204, 206, and/or 208.

FIG. 15 is a diagram of wearable ISF sensing module 100 positioned on the user's skin 216 with microneedles 114 inserted into a dermis 218 of the user's skin 216. During operation, the user attaches wearable ISF sensing module 100 to their skin 216 such that microneedles 114 puncture and penetrate an epidermis 220 and dermis 218 of user's skin 216. Specifically, sensors 144 are embedded in dermis 218 of user's skin 216 when microneedles 114 are inserted into user's skin 216. Once inserted into dermis 218, ISF from the vicinity of the cells surrounding microneedles 114 flows into channel 210 and contacts electrodes 200, 202, 204, 206, and/or 208. Electrodes 200, 202, 204, 206, and/or 208 detect and measure at least one specific biomarker within the ISF. Electrodes 200, 202, 204, 206, and/or 208 then send an electrical signal to on-patch control module 172 and/or off-patch control module 174. On-patch control module 172 and/or off-patch control module 174 may analyze the data from electrodes 200, 202, 204, 206, and/or 208 and/or may transmit the data from electrodes 200, 202, 204, 206, and/or 208 to the computing device. The user and/or the medical professional may then analyze the data to determine if further medical care is required.

Because sensors 144 are embedded in dermis 218, the ISF that flows to sensors 144 is continually replenished and mechanical mechanisms to replenish the ISF are not required to enable wearable ISF sensing module 100 to operate as described herein. Additionally, embedding sensors 144 in dermis 218 enables wearable ISF sensing module 100 to detect biomarkers without drawing fluid out of the body of the user, reducing the cleanup and disposal of bodily fluids as the result of biomarker detection and measurement, and reducing the equipment required to measure biomarkers within the ISF. Moreover, because the pressure of the ISF causes the ISF to flow into channel 210, a fluid motive device, such as a vacuum pump, is not required to continually replenish the ISF flowing to electrodes 200, 202, 204, 206, and/or 208.

The embodiments described herein include systems and methods for wearable ISF sensing devices. The device includes a plurality of layers laminated together to form a patch component and at least one microneedle extending from the patch component. The layers include a first patch layer, a first adhesive layer, an electronics module component layer, a second adhesive layer, and a second patch layer. The first and second patch layers structurally support the electronics module component layer, and the first and second adhesive layers laminate the first and second patch layers to the electronics module component layer. Lamination of the layers ensures that the microneedles remain intact as they are inserted into the skin of the user. As such, the microneedles of the wearable ISF sensing devices described herein are structurally stronger than previous microneedles and remain intact as they monitor biomarkers within the user's ISF.

The microneedle includes a microneedle base, a body, and a tip. The microneedle has a substantially flat or planar profile and a slot is defined within the body of the microneedle. The slot has a first opening and a second opening. The electronics module component layer includes a sensor positioned over the second opening such that the sensor and the slot define a channel that channels ISF to the sensor. The microneedle and the sensor are inserted into the user's skin such that the sensor is positioned within the dermis of the user's skin. The ISF flows into the channel from adjacent tissue, and the sensor detects and measures biomarkers within the ISF. The user, observer, trainer, coach, and/or a medical professional may then monitor a medical condition or performance/readiness metric based on the data collected by the sensor.

Because the sensors are embedded in the dermis, the ISF that flows to the sensors is continually equilibrated with the surrounding ISF without requiring mechanical mechanisms to replenish the ISF. Additionally, embedding the sensors in the dermis enables the wearable ISF sensing device to detect biomarkers without drawing fluid out of the body of the user, reducing the cleanup and disposal of bodily fluids as the result of biomarker detection and measurement. Moreover, capillary pressure and the compressive force generated during insertion will cause ISF to flow into the channel such that a fluid motive device, such as a vacuum pump or mechanical compression, is not required to replenish the ISF at the sensors. After insertion, passive diffusion from the ISF and equilibration with the ISF inside the channel replenishes the ISF at the sensors. Thus, the wearable ISF sensing device enables detection of biomarkers while reducing cleanup and disposal of bodily fluids and reducing the equipment required to measure biomarkers within the ISF. Additionally, directly measuring ISF in the interstitial space reduces the lag time between changes in biomarker concentration and read-out.

An exemplary technical effect of the methods, systems, and apparatus described herein includes at least one of: (a) detecting biomarkers within ISF; (b) reducing cleanup and disposal of bodily fluids associated with biomarker detection; (c) reducing the equipment required to measure biomarkers within the ISF; and (d) reducing the error associated with detection of biomarkers within ISF.

Exemplary embodiments for wearable ISF sensing devices are described herein. The systems and methods are not limited to the specific embodiments described herein, but rather, components of systems and/or steps of the methods may be utilized independently and separately from other components and/or steps described herein. For example, the methods may also be used in combination with other systems, and are not limited to practice with only the systems described herein. Rather, the exemplary embodiment can be implemented and utilized in connection with many other systems.

Although specific features of various embodiments of the disclosure may be shown in some drawings and not in others, this is for convenience only. In accordance with the principles of the disclosure, any feature of a drawing may be referenced and/or claimed in combination with any feature of any other drawing.

This written description uses examples to disclose the embodiments, including the best mode, and also to enable any person skilled in the art to practice the embodiments, including making and using any devices or systems and performing any incorporated methods. The patentable scope of the disclosure is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. A wearable interstitial fluid (ISF) sensing device (100) comprising:
a first patch layer (102); and
an electronics module component layer (106) laminated to said first patch layer (102) to form a patch component (112) and a plurality of microneedles (114) extending from said patch component (112), said electronics module component layer (106) comprising a substrate (168) and at least one sensor (144) positioned on said substrate (168), each microneedle of said plurality of microneedles (114) comprising:
a microneedle base (146) extending from said patch component (112);
a body (148) extending from said microneedle base (146), said body (148) defining a slot (160) extending through said first patch layer (102) of said body (148), the slot (160) defining a first opening (212) and a second opening (214), said at least one sensor (144) positioned over the second opening (214), wherein the first opening (212), the slot (160), and said at least one sensor (144) define a channel (210) exposed to the environment; and
a tip (150) extending from said body (148), wherein the channel (210) is configured to channel (210) ISF to said at least one sensor (144).

2. The wearable ISF sensing device (100) of Claim 1 further comprising a second patch layer (110) laminated to said first patch layer (102) and said electronics module component layer (106), wherein said electronics module component layer (106) is positioned between said first patch layer (102) and said second patch layer (110), and wherein said first patch layer (102) and said second patch layer (110) structurally support said electronics module component layer (106).

3. The wearable ISF sensing device (100) of Claim 2 further comprising a first adhesive layer (104) positioned between said first patch layer (102) and said electronics module component layer (106), wherein said first adhesive layer (104) laminates said first patch layer (102) to said electronics module component layer (106).

4. The wearable ISF sensing device (100) of Claim 3, wherein said first adhesive layer (104) comprises a sheet of a curable adhesive.

5. The wearable ISF sensing device (100) of Claim 3, wherein said first adhesive layer (104) comprises a pressure sensitive adhesive.

6. The wearable ISF sensing device (100) of Claim 2 further comprising a second adhesive layer (108) positioned between said second patch layer (110) and said electronics module component layer (106), wherein said second adhesive layer (108) laminates said second patch layer (110) to said electronics module component layer (106).

7. The wearable ISF sensing device (100) of Claim 6, wherein said second adhesive layer (108) comprises a sheet of a curable adhesive.

8. The wearable ISF sensing device (100) of Claim 6, wherein said second adhesive layer (108) comprises a pressure sensitive adhesive.

9. The wearable ISF sensing device (100) of Claim 2, wherein said slot (160) comprises a first slot (160), wherein said body (148) defines a second slot (160) extending through said second patch layer (110) of said body (148), wherein the second slot (160) defines a third opening and a fourth opening, wherein said at least one sensor (144) comprises at least one first sensor (109), wherein said electronics module component layer (106) further comprises at least one second sensor (192) positioned on said substrate (168), wherein said at least one sensor (144) is positioned over the fourth opening, and wherein the third opening, the second slot (160), and said at least one second sensor (192) define a second channel (210) exposed to the environment.

10. The wearable ISF sensing device (100) of Claim 1, wherein said microneedle base (146) comprises a pliable material.

11. The wearable ISF sensing device (100) of Claim 1, wherein said electronics module component comprises an on-patch control module (172) configured to enable wireless communication with a mobile computing device.

12. The wearable ISF sensing device (100) of Claim 1, wherein said electronics module component further comprises an on-patch control module (172) and an off-patch control module (174).

13. The wearable ISF sensing device (100) of Claim 12 further comprising a cable communicatively coupling said at least one sensor (144) and said on-patch control module (172) to said off-patch control module (174).

14. The wearable ISF sensing device (100) of Claim 12, wherein said on-patch control module comprises an analog-to-digital conversion signal module component coupled to said base.

15. A method of detecting a biomarker within the interstitial fluid (ISF) of a user, the method comprising:
adhering a wearable ISF sensing device (100) to the user's skin, the wearable ISF sensing device (100) including at least one microneedle (114) and at least one sensor (144) positioned within the microneedle, the at least one microneedle (114) including a microneedle base (146), a body (148) extending from the microneedle base (146), and a tip (150) extending from the body (148), the body (148) defining a slot (160) extending through the body (148), the slot (160) defining a first opening (212) and a second opening (214), the at least one sensor (144) positioned over the second opening (214), wherein the first opening (212), the slot (160), and the at least one sensor (144) define a channel (210);
channeling the ISF into the channel (210) to the at least one sensor (144); and
detecting at least one biomarker with the at least one sensor (144).
